# EUROPEAN PATENT SPECIFICATION

(11) **EP 2 881 092 B1**
(45) Date of publication and mention of the grant of the patent: **04.03.2020**
(21) Application number: 14194741.6
(22) Date of filing: 25.11.2014
(51) Int. Cl.: A61F 7/02, A61F 7/00, A61F 7/08

(54) **Thermotherapeutic pad and method of manufacturing a thermotherapeutic pad**
Thermotherapeutisches Pad und Verfahren zur Herstellung eines thermotherapeutischen Pads
Compresse thermothérapeutique et procédé de fabrication associé

(30) Priority: 03.12.2013 US 201361911305 P; 20.12.2013 US 201314136478
(43) Date of publication of application: 10.06.2015
(73) Proprietor: Rapid Aid Corp., Mississauga Ontario L5L 5Z3 (CA)
(72) Inventor: Whitely, Jeffrey Thomas, Mississauga, Ontario L5L 5Z3 (CA)
(74) Representative: dompatent von Kreisler Selting Werner - Partnerschaft von Patent- und Rechtsanwälten mbB

(56) References cited:
- EP-A1- 0 967 944
- EP-A1- 1 997 460
- JP-A- H0 838 538
- US-A- 4 920 964
- US-A- 5 415 624
- US-A- 6 083 254
- US-B1- 6 830 582

## Description

### FIELD

The specification relates generally to pads and packs for thermal therapy, and specifically to a thermotherapeutic pad and a method of manufacturing a thermotherapeutic pad.

### BACKGROUND

Many typical thermotherapeutic pads (also referred to herein as "thermotherapeutic packs") include at least some form of vinyl material or fabric. However, vinyl has a number of disadvantages. Vinyl, particularly when exposed to cold temperatures, tends to be prone to cracking (i.e. exhibits low crack resistance). This can be problematic for thermotherapeutic pads being that are used for cold therapies since the composition retained inside the pad or pack may leak if the vinyl material cracks.

Furthermore, vinyl material may require radio-frequency (RF) heat sealing, which can be costly. Over their lifetime, pads having a substantial amount of vinyl may puff up with air, thereby reducing their thermal effectiveness. Vinyl material may also become unsuitably softened in the **microwave**, a common and convenient method of heating up products, including thermotherapeutic pad.

Furthermore, some typical thermotherapeutic pads require a user to hold the thermotherapeutic pads in place against a body part during thermal therapy. This can be tiresome, particularly if the thermal therapy requires multiple sessions, and difficult, particularly if the user has low dexterity or strength or if the pad is large. US patent no. 4,920,964 and US patent no. 6,083,254 disclose examples of thermal packs for both heating and cooling comprising an inner polyethylene layer and an outer Nylon layer. US patent no. 6,830,582 discloses a re-sealable closing mechanism for a hot/cold pouch, the pouch having an outer layer, an intermediate Nylon layer, and an inner polyethylene layer, the pouch fillable with hot or cold material to provide hot or cold therapy.

Nevertheless, the prior art suffers for lack of a thermotherapeutic pads that can be effectively used for both hot and cold therapy and that are also convenient and simple to use.

### SUMMARY

The specification relates to pads and packs for thermal therapy and methods of manufacturing the same. Such thermotherapeutic pads include a layer or multiple layers of nylon material rather than vinyl.

According to one aspect of the specification, there is provided a thermotherapeutic pad comprised of a pouch formed from at least one laminate sheet. The at least one laminate sheet includes an outer polyester layer, an intermediate nylon layer and an inner polyethylene layer. The pouch is permanently sealed closed. A thermotherapeutic composition **comprising a gel material capable of being heated and cooled** is pre-loaded into the pouch. The thermotherapeutic composition is for providing hot therapy or cold therapy when the pouch is applied against a body part.

According to another aspect of the specification, there is provided a thermotherapeutic pad comprised of a pouch formed from at least one laminate sheet. The at least one laminate sheet includes an outer polyester layer of a warp-knitted polyester fabric, an intermediate nylon layer and an inner polyethylene layer. The pouch is permanently sealed closed by one or more heat bonded seams. A thermotherapeutic composition is pre-loaded into the pouch. The thermotherapeutic composition is for providing hot therapy or cold therapy when the pouch is applied against a body part. A first adhesive is applied to one or more of the intermediate nylon layer and the inner polyethylene layer. A second adhesive polyethylene adhesive surface bonds the outer polyester layer to the intermediate nylon layer. At least one strap having a fixed end is attached to a portion of the pouch. The at least one strap has a length sized to wrap around the body part to undergo thermal therapy to retain the pouch against the body part. The at least one strap also has a fastener at a free end opposite the fixed end. The fastener is configured to engage the at least one strap or the pouch. At least one buckle for anchoring the at least one strap is attached to another portion of the pouch. The at least one strap is anchored by the at least one buckle when threaded through and overlapping with the buckle.

According to another aspect of the specification, a method of manufacturing a thermotherapeutic pad is provided. The method comprises permanently sealing at least one laminate sheet to form a pouch having an open top. The at least one laminate sheet includes an outer polyester layer, an intermediate nylon layer and an inner polyethylene layer. The method also includes loading the pouch with a thermotherapeutic composition for providing, when the pouch is applied against a body part, hot therapy or cold therapy, and closing the pouch by permanently sealing the open top.

According to another aspect of the specification, another method of manufacturing a thermotherapeutic pad is provided. The method comprises forming at least one laminate sheet including an outer polyester layer, an intermediate nylon layer and an inner polyethylene layer. The at least one laminate sheet is formed by adhering the intermediate nylon layer to the inner polyethylene layer, surface bonding the outer polyester layer to the intermediate nylon layer, and heat curing the at least one laminate sheet. The method also includes permanently sealing at least one laminate sheet to form a pouch having an open top and includes loading the pouch with a thermotherapeutic composition for providing, when the pouch is applied against a body part, hot therapy or cold therapy, and closing the pouch by permanently sealing, by heat bonding, the open top. The method includes attaching at least one strap at a fixed end to a portion of the pouch, the at least one strap having a length sized to wrap around the body part to undergo thermal therapy to retain the pouch against the body part, the at least one strap having a fastener at a free end opposite the fixed end, the fastener configured to engage the at least one strap or the pouch. The method further includes attaching to another portion of the pouch, at least one buckle for anchoring the at least one strap, the at least one strap anchored by the at least one buckle when threading the at least one strap through and overlapping the at least one buckle. The invention is defined by the appended independent claims; preferred embodiments are given in the dependent claims.

### BRIEF DESCRIPTIONS OF THE FIGURES

For a better understanding of the various implementations described herein and to show more clearly how they may be carried into effect, reference will now be made, by way of example only, to the accompanying drawings in which:
FIG. 1 depicts a thermotherapeutic pad, according to non-limiting implementations.
FIG. 2 depicts the at least one laminate sheet of the thermotherapeutic pad of FIG. 1, according to non-limiting implementations.
FIG. 3 depicts a close-up view of corner "A" of the thermotherapeutic pad of FIG. 1, according to non-limiting implementations.
FIG. 4 depicts a perspective view of the thermotherapeutic pad of FIG. 1, according to non-limiting implementations.
FIG. 5 shows a thermotherapeutic pad having a strap and a buckle, according to non-limiting implementations.
FIG. 6 shows a close-up view of a stitched fixed end of the strap of the thermotherapeutic pad shown in FIG. 5, according to non-limiting implementations.
FIG. 7 shows a close-up view of a stitched fixed end of the strap of the thermotherapeutic pad shown in FIG. 5, according to non-limiting implementations.
FIG. 8 shows a close-up view of a stitched attachment point of the buckle of the thermotherapeutic pad shown in FIG. 5, according to non-limiting implementations.
FIG. 9 depicts a thermotherapeutic pad having a strap with a fastener as a free end and a buckle, according to non-limiting implementations.
FIG. 10 depicts a close-up view of the fastener shown in FIG. 9.
FIG. 11 depicts a thermotherapeutic pad, according to non-limiting implementations, wrapped around an arm.
FIG. 12 depicts a thermotherapeutic pad with a strap threaded through a buckle, according to non-limiting implementations.
FIG. 13 depicts a thermotherapeutic pad with a strap threaded through a buckle, according to non-limiting implementations.
FIG. 14 depicts a close-up view of a strap threaded through a buckle, according to non-limiting implementations.
FIG. 15 depicts a thermotherapeutic pad with a strap threaded through a buckle and showing a fastener, according to non-limiting implementations.
FIG. 16 depicts a flowchart of a method of manufacturing a thermotherapeutic pad, according to non-limiting implementations.

### DETAILED DESCRIPTION

Described herein is a pad, referred herein as "a thermotherapeutic pad", capable of providing thermotherapy (also referred to as "thermal therapy") in the form hot therapy, cold therapy or both. The thermotherapeutic pad can also be reused. Also described herein is a method of manufacturing such a thermotherapeutic pad. The thermotherapeutic pad has at least one layer of nylon material.

In contrast, many typical thermotherapeutic pads include at least some form of vinyl material. However, vinyl has a number of disadvantages. Vinyl, particularly when exposed to cold temperatures, tends to be prone to cracking (i.e. exhibits low crack resistance). This can be problematic for thermotherapeutic pads that are being used for cold therapies since the composition retained inside the pad may leak if the vinyl material cracks.

Furthermore, vinyl material may require radio-frequency (RF) heat sealing, which can be costly. Also, vinyl may permit the thermotherapeutic pad to puff up with air over its lifetime, thereby reducing the pad or pack's heat transfer effectiveness. Vinyl material may also not be suitable for heating in the **microwave**, a common and convenient method of heating up products, including thermotherapeutic pad.

Nylon material can provide certain advantages over vinyl material. For example, nylon material can be more crack resistant than vinyl material, which allows for the thermotherapeutic pad to be exposed to cooler temperatures, including temperatures suitable for cold therapy. Nylon is also better suited for use in microwave ovens. As a result, a user of the thermotherapeutic pad would have the option of heating the thermotherapeutic pad in a manner that is likely more convenient and faster than other methods.

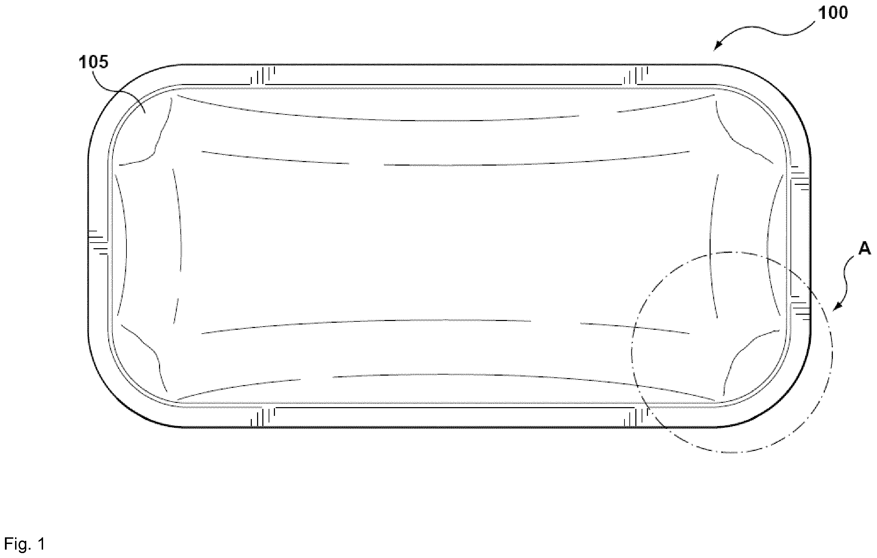

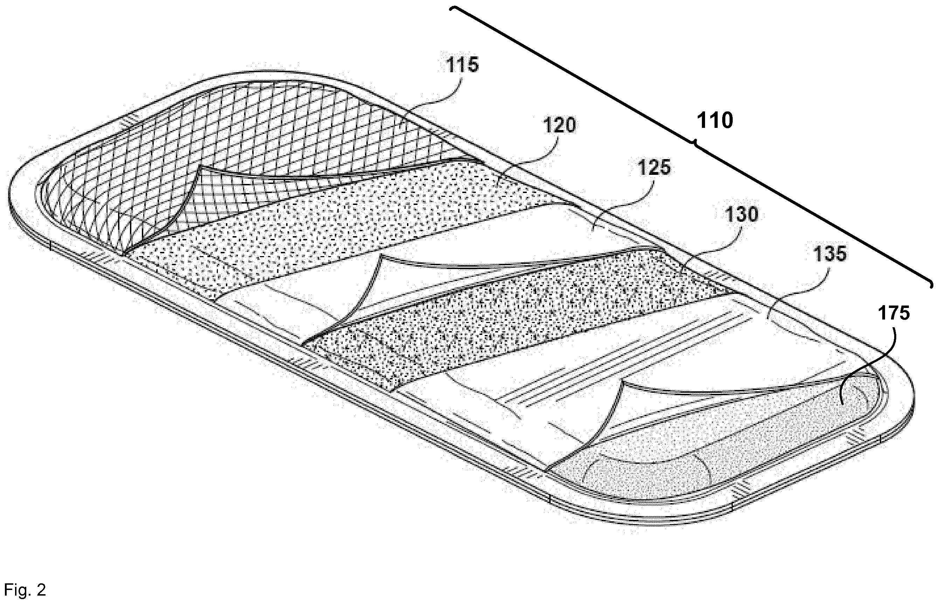

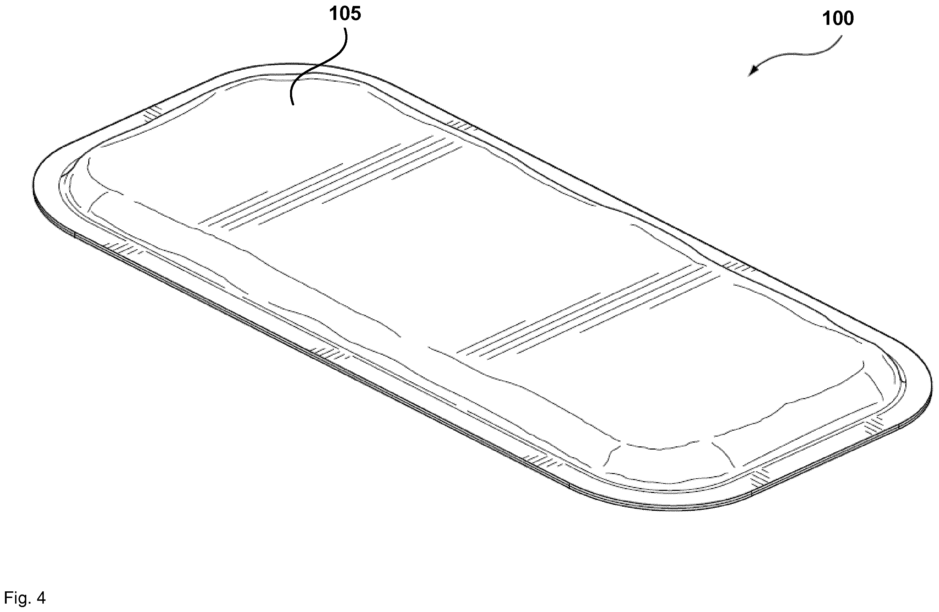

FIG. 1 shows thermotherapeutic pad 100. The thermotherapeutic pad 100 can be applied to a portion of a person's body or body part, such as an arm, leg, neck, abdomen, etc. Thermotherapeutic pad 100 comprises a pouch 105 formed from at least one laminate sheet 110 (shown in FIG. 2). A thermotherapeutic composition (not shown) is pre-loaded into the pouch 105. When the pouch 105 is applied against a body part, or a portion of a body part, the thermotherapeutic composition can provide hot therapy when the thermotherapeutic composition is heated and cold therapy when the thermotherapeutic composition is cooled. FIG. 4 depicts thermotherapeutic pad 100 from another perspective.

As shown in FIG. 2, at least one laminate sheet 110 includes an outer polyester layer 115, an intermediate nylon layer 125 and an inner polyethylene layer 135. Outer polyester layer 115 forms at least part of an exterior surface of the thermotherapeutic pad 100 that is to be applied against a body part.

According to some implementations, an adhesive, represented by adhesive layer 130 in FIG. 2, is applied to one or more of the intermediate nylon layer 125 and the inner polyethylene layer 135. According to some implementations, adhesive layer 130 comprises a polyethylene adhesive.

According to some implementations, an additional adhesive, represented by additional polyethylene adhesive layer 120 in FIG. 2, is applied to one or more of outer polyester layer 115 and intermediate nylon layer 125 to surface bond outer polyester layer 115 to intermediate nylon layer 125.

According to some implementations, the at least one laminate sheet 110 is heat cured. According to some implementations, the at least one laminate sheet 110 has a thickness of approximately 2.5 thousandths of an inch **(approximately 0.00635 centimeters).**

According to some implementations, outer polyester layer 115 comprises a warp-knitted polyester fabric. According to some implementations, the warp-knitted polyester fabric has a weight of approximately 120 grams per square meter.

A warp-knitted polyester fabric can provide certain benefits. Since outer polyester layer 115 forms at least part of an exterior surface of the thermotherapeutic pad 100 that is to be applied against a body part, its soft texture can be more comfortable to the user than another synthetic, non-textured material. According to some implementations, tiny air pockets form within the warp-knitted polyester fabric of outer polyester layer 115. These air pockets can provide some insulation from the hot or cold temperatures of the pouch 105 when thermotherapeutic pad 100 is being used for hot or cold therapy. For example, if thermotherapeutic pad 100 has been heated to a temperature that is not comfortable for a user, the air pockets in outer polyester layer 115 can regulate the heat emanating from pouch 105 to minimize the intensity of heat actually experienced by the user when the thermotherapeutic pad 100 is applied against a body part. Furthermore, if thermotherapeutic pad 100 has been heated or cooled unevenly, the air pockets of outer polyester layer 115 may help minimize any "hot spots" or "cold spot" as experienced by the user when the thermotherapeutic pad 100 is applied against a body part. Furthermore, the warp-knitted polyester fabric may allow a fastener of any strap or straps used to retain pouch 105 against a body part, as discussed below, to be secured to pouch 105.

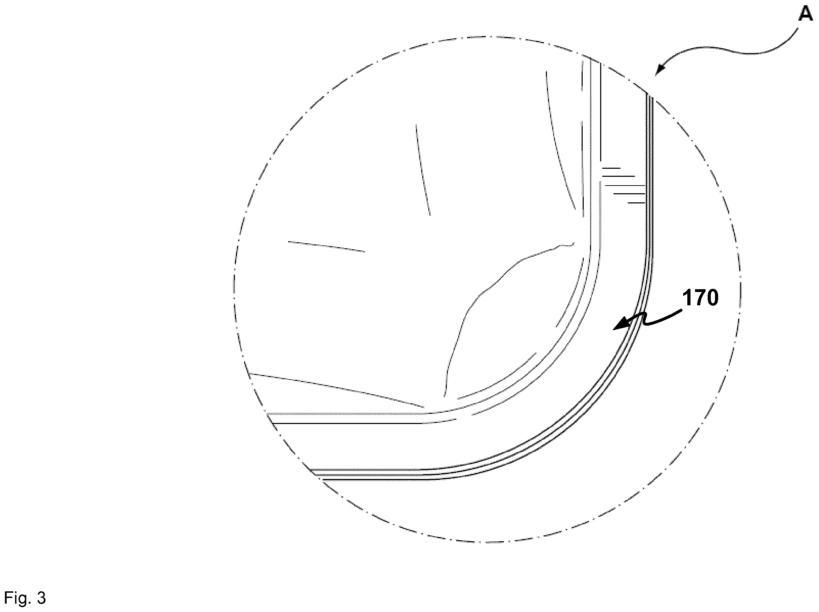

Pouch 105 is permanently sealed closed. Hence pouch 105 cannot be re-filled or re-sealed by the user. According to some implementations, pouch 105 is permanently sealed closed by one or more heat bonded seams. FIG. 3 depicts a corner of pouch 105 (corner "A" of FIG. 1) in which a seam 170 formed by the at least one laminate sheet 110 has been heat sealed.

Pouch 105 can be formed from the at least one laminate sheet 110 in a number of ways. According to some examples, pouch 105 is formed by folding one laminate sheet in half and permanently sealing closed perimeter edges of the one laminate sheet. According to some implementations, pouch 105 is formed from two laminate sheets in which the perimeter edged of the two laminate sheets are permanently sealed together (closed).

As stated above, a thermotherapeutic composition 175 is pre-loaded into the pouch 105. According to the invention, the thermotherapeutic composition comprises a gel material capable of being heated and cooled. For example, the thermotherapeutic composition comprises a gel material that is capable of being heated in a microwave and cooled in a freezer.

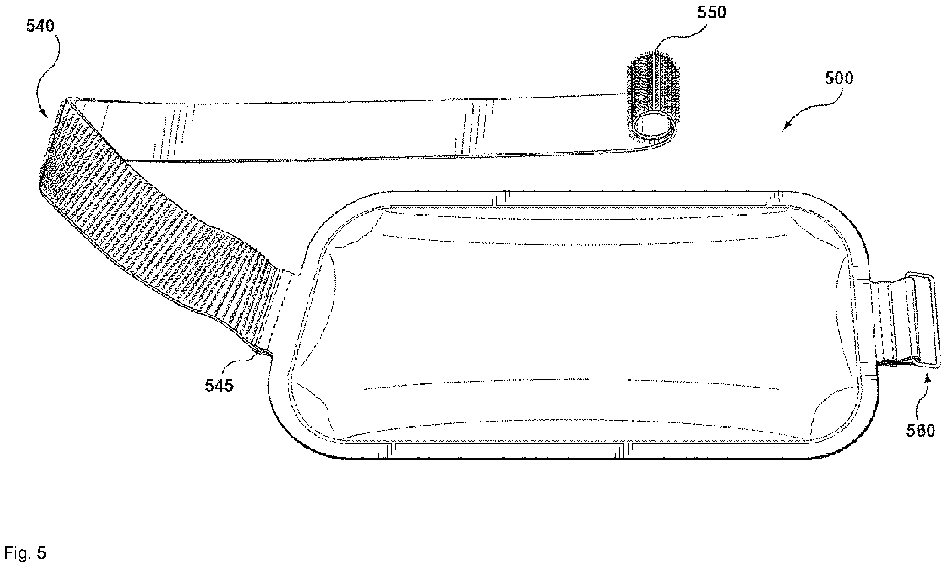

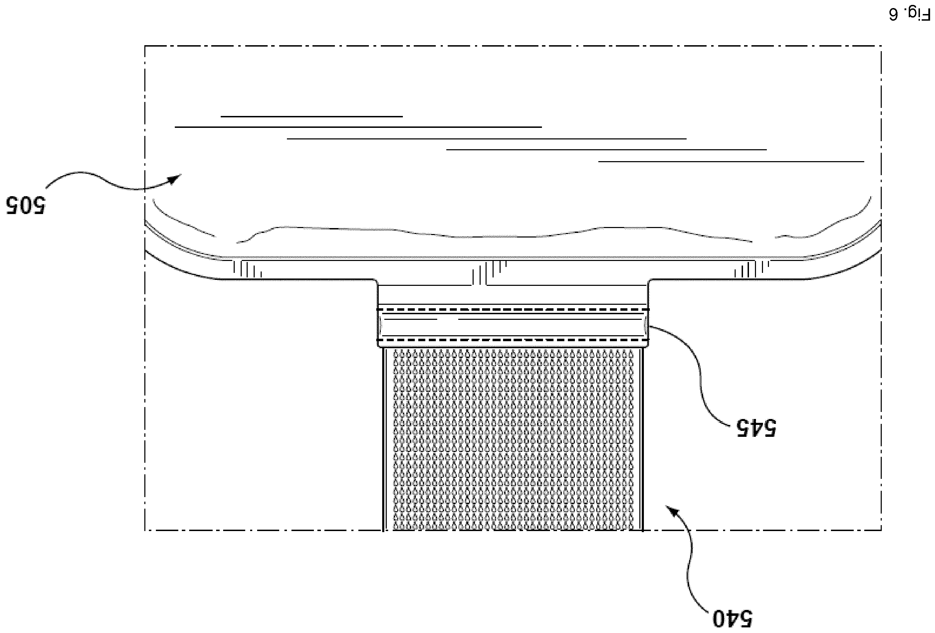

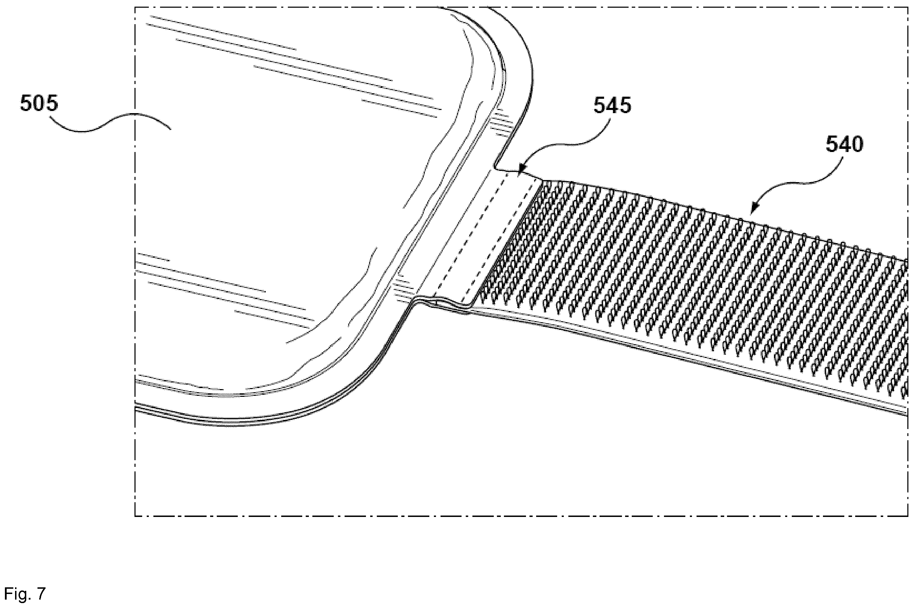

Attention is now directed to FIG. 5, which depicts thermotherapeutic pad 500, in which like elements are denoted by like or similar numbers to FIGS. 1 to 4, however starting with a "5" rather than a "1". Thermotherapeutic pad 500 has at least one strap 540 having a fixed end 545 affixed to a portion of pouch 505. According to some implementations, strap 540 is affixed to a portion of pouch 505 by one or more of stitching (as, for example, shown in FIGS. 6 and 7) and heat sealing. Strap 540 has a length sized to wrap around the body part to undergo thermal therapy to retain the pouch 105 against the body part. According to some implementations, thermotherapeutic pad 500 has more than one strap 540.

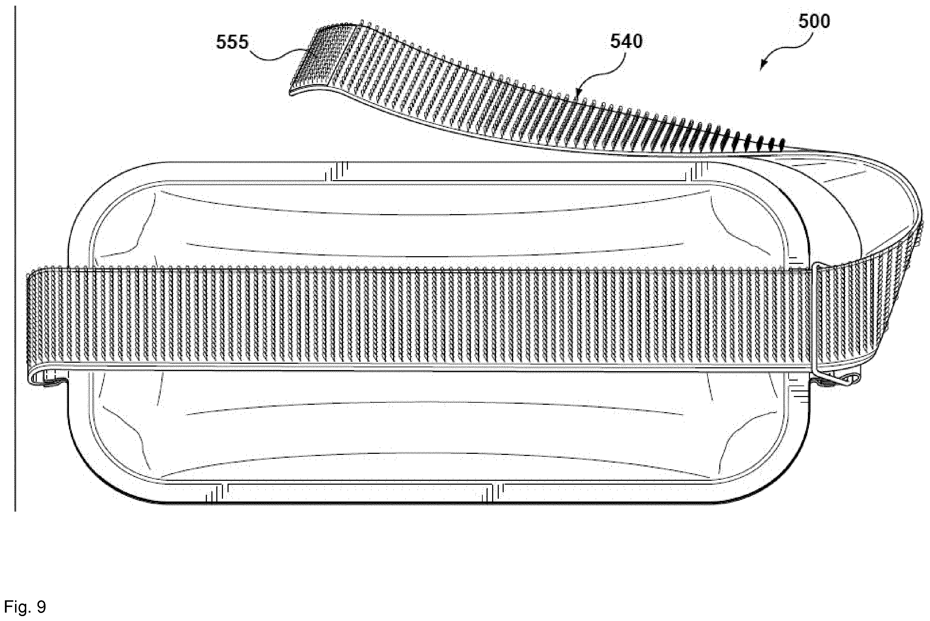

According to some implementations, strap 540 has a fastener 555(as, for example, shown in FIG. 9) at a free end 550 opposite the fixed end, the fastener configured to engage the at least one strap 540 or the pouch 505. According to some implementations, fastener 555 comprises one or more of a hook side of a hook-and-loop fastener (as, for example, shown in FIG. 9) and a removable adhesive panel. According to some implementations, at least a portion of strap 540 is configured to engage with fastener 555. For example, as shown in FIGS. 5 to 7, according to some implementations at least a portion of strap 540 comprises loop material to engage with a hook side of fastener 555.

According to some implementations, as depicted in FIG. 5, thermotherapeutic pad 500 includes at least one buckle 560 affixed to another portion of the pouch 505 for anchoring strap 540. Buckle 560 is sized to receive strap 540. By threading or passing strap 540 through buckle 560 (as, for example, shown in FIG. 9), strap 540 is anchored by buckle 560. Strap 540 can then be pulled to cinch or secure pouch 505 against a body part. Buckle 560 provides a mechanical advantage that approximately doubles the force applied by the user to the strap 540. This may help users with lesser dexterity or strength apply thermotherapeutic pad 500 against the desired body part.

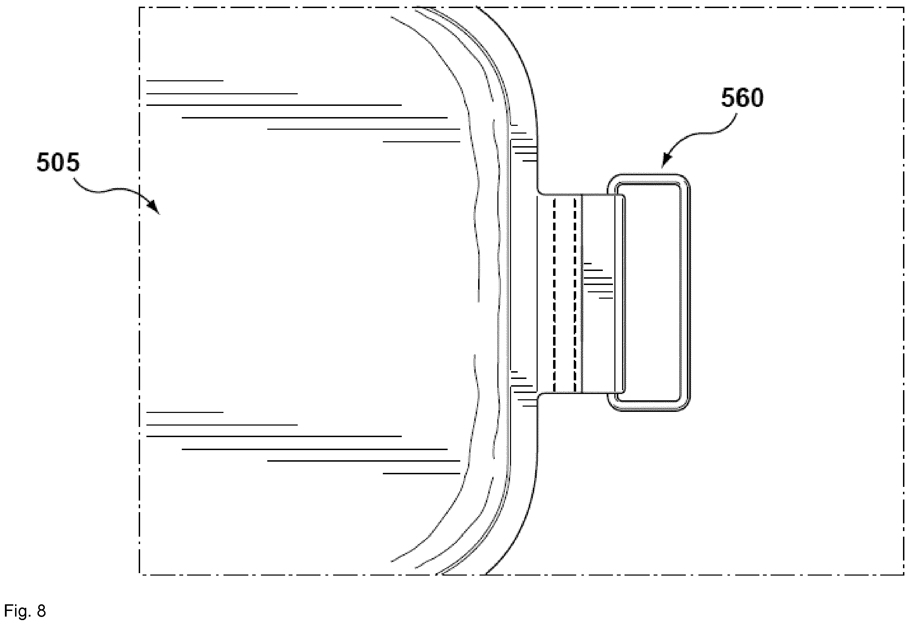

According to some implementations, buckle 560 is affixed to another portion of pouch 505 by one or more of stitching (as, for example, shown in FIG. 8) and heat sealing.

Buckle 560 can be fabricated from a variety of materials. For example, the buckle 560 can be fabricated plastic, metal, or a combination of such. Using plastic for the buckle 560 is advantageous if the pad 500 is to be used in a microwave oven, and further generally helps prevent the buckle from getting too hot or too cold and potentially injuring the user.

According to some implementations, thermotherapeutic pad 500 includes more than one buckle 560.

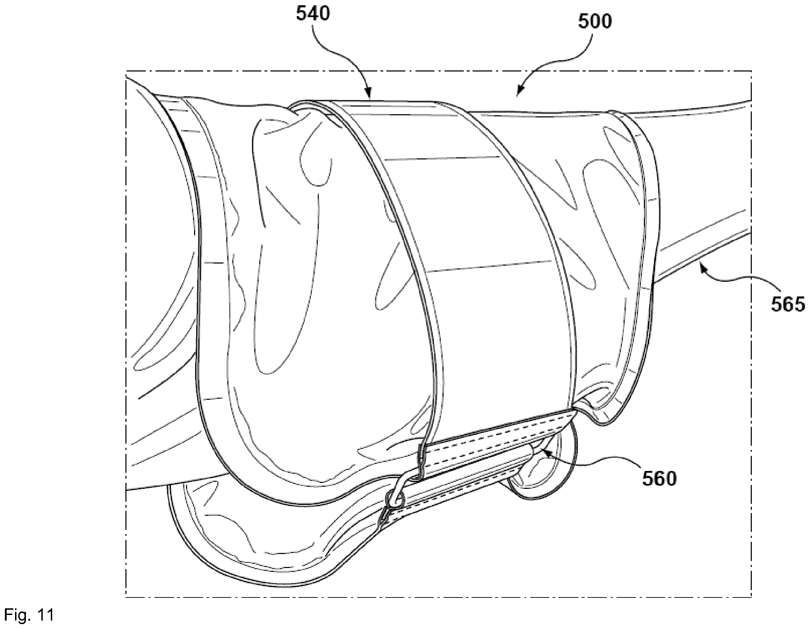

Attention is now directed to FIG. 11 which depicts thermotherapeutic pad 500 wrapped around arm 565. As shown in FIG. 11, strap 540 is threaded through and overlaps buckle 560 to wrap around pouch 505, and pulled to cinch pouch 505 against arm 565. Strap 540 can be further secured by engaging fastener 555 (not shown) with another portion of strap 540 or pouch 505.

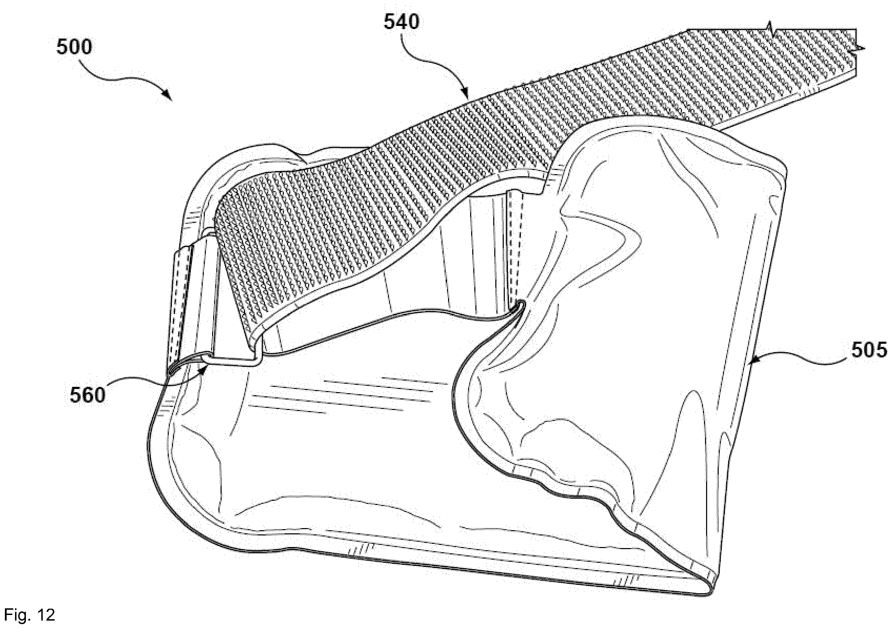

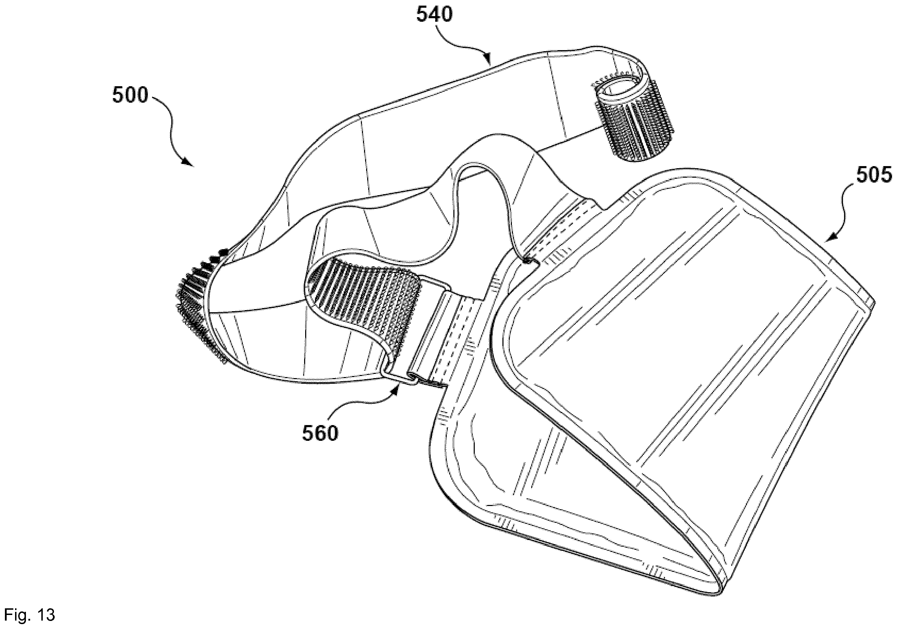

According to some implementations, at least pouch 505 of thermotherapeutic pad 500 can be twisted, bent or otherwise contorted to adapt pouch 505 for application against at least a portion of a body part. For example, FIG. 12 depicts thermotherapeutic pad 500 with strap 540 threaded through buckle 560 and in which a portion of the pouch 505 is folded over. FIG. 13 depicts thermotherapeutic pad 500 with strap 540 threaded through buckle 560 and in which pouch 505 is folded over approximately in half.

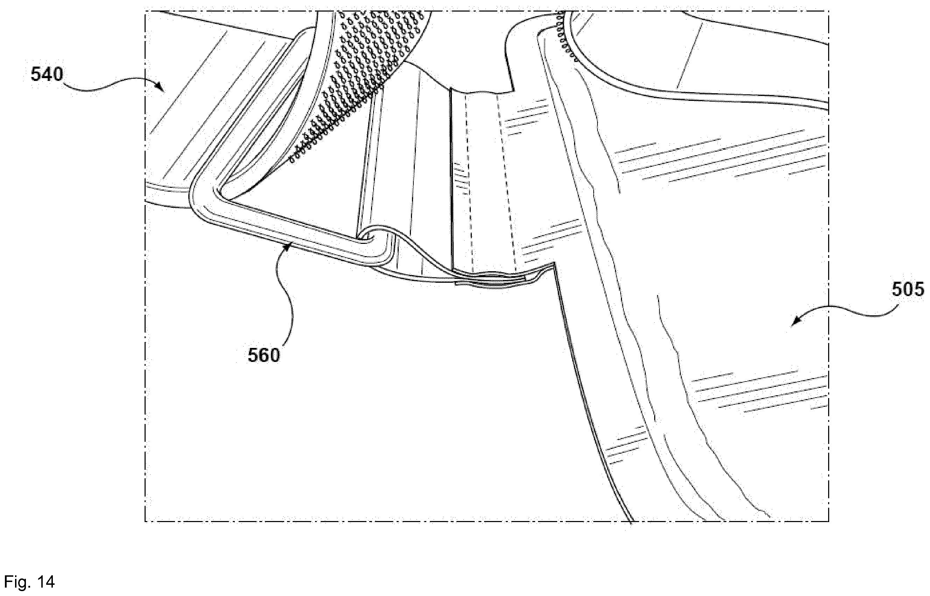

FIG. 14 depicts a close-up view of strap 540 threaded through buckle 560.

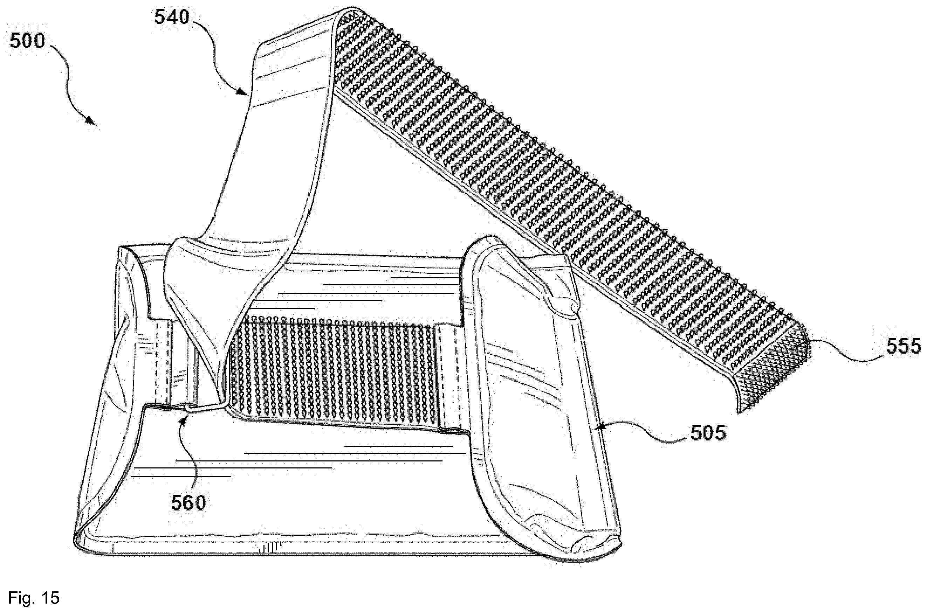

FIG. 15 depicts thermotherapeutic pad 500 with strap 540 threaded through buckle 560 and showing fastener 555.

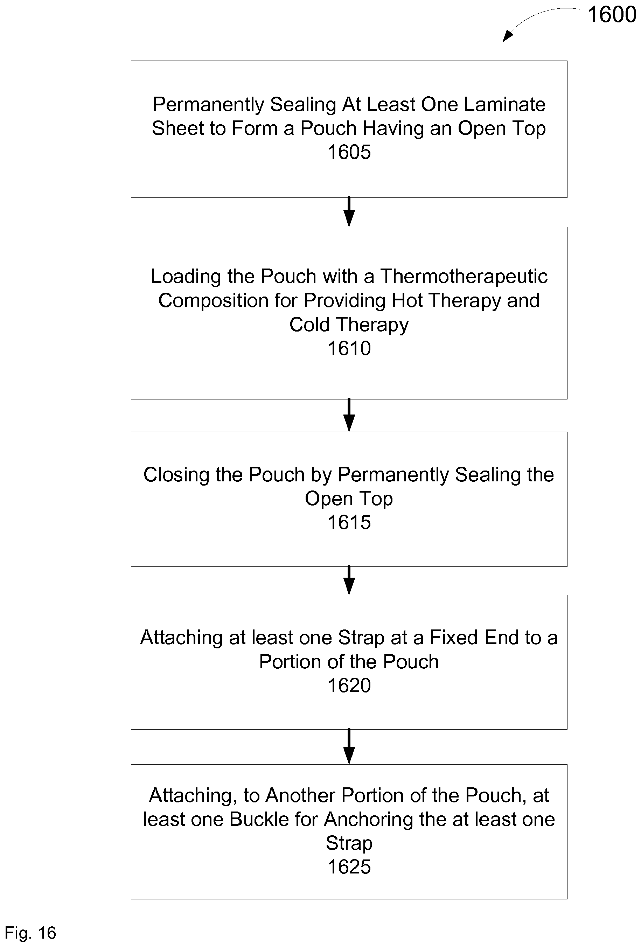

Attention is now directed to Fig. 16 which depicts a flowchart of a method 1600 of manufacturing a thermotherapeutic pad, according to non-limiting implementations. However, it is to be understood that method 1600 can be varied, and need not work exactly as discussed herein in conjunction with each other, and that such variations are within the scope of present implementations.

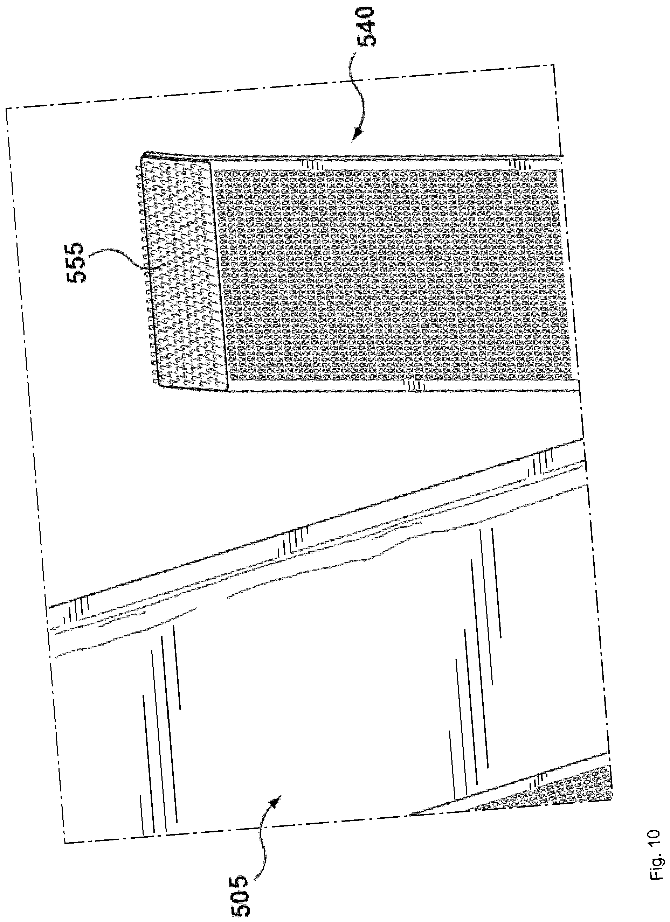

It is to be emphasized, however, that method 1600 need not be performed in the exact sequence as shown, unless otherwise indicated. Further, method 1600 is described with reference to FIGS. 1 to 15.

At block 1605, at least one laminate sheet 110 is permanently sealed to form a pouch having an open top, the at least one laminate sheet 110 including an outer polyester layer 115, an intermediate nylon layer 125 and an inner polyethylene layer 135. According to some implementations, the at least one laminate sheet 110 is formed by adhering the intermediate nylon layer 125 to the inner polyethylene layer 135, and surface bonding the outer polyester layer 115 to the intermediate nylon layer 125. According to some implementations, forming of the at least one laminate sheet 110 further comprises heat curing the at least one laminate sheet 110. According to some implementations, permanently sealing the at least one laminate sheet 110 to form a pouch having an open top comprises heat bonding.

At block 1610, the pouch having an open top is loaded with a thermotherapeutic composition for providing, when the pouch is applied against a body part, hot therapy and cold therapy.

At block 1615, the pouch 105 is closed by permanently sealing the open top. According to some implementations, permanently sealing the open top to close the pouch comprises heat bonding.

According to some implementations, method 1600 further comprises, at block 1620, attaching at least one strap 540 at a fixed end 545 to a portion of the pouch 505. As described above, the at least one strap 540 has a length sized to wrap around the body part to undergo thermal therapy to retain the pouch 505 against the body part. The at least one strap 540 also has a fastener 555 at a free end opposite the fixed end 545. Fastener 555 is configured to engage the at least one strap 540 or the pouch 505. According to some implementations, attaching the at least one strap 540 comprises performing one or more of: stitching and heat sealing.

According to some implementations, method 1600 further comprises, at block 1625, attaching to another portion of the pouch 505, at least one buckle 560 for anchoring the at least one strap 540. As stated above, the at least one strap 540 is anchored by the at least one buckle 560 when threaded or passed through to overlap the at least one buckle 560. According to some implementations, attaching the at least one buckle 560 comprises performing one or more of: stitching and heat sealing.

Persons skilled in the art will appreciate that there are yet more alternative implementations and modifications possible, and that the above examples are only illustrations of one or more implementations. The scope, therefore, is only to be limited by the claims appended hereto.

## Claims

1. A **re-usable** thermotherapeutic pad **(100, 500)** comprising:
a pouch **(105, 505)** formed from at least one laminate sheet **(110),**
the at least one laminate sheet **(110)** including an outer polyester layer **(115),** an intermediate nylon layer **(125)** and an inner polyethylene layer **(135),** and
the pouch **(105, 505)** being permanently sealed closed; and
a thermotherapeutic composition **(175)** pre-loaded into the pouch **(105, 505),** the thermotherapeutic composition **(175) comprising a gel material capable of being heated and cooled** for providing, when the pouch **(105, 505)** is applied against a body part, hot therapy when the thermotherapeutic composition **(175)** is heated and cold therapy when the thermotherapeutic composition **(175)** is cooled.

2. The thermotherapeutic pad **(100, 500)** of claim 1, further comprising an adhesive **(120, 130)** applied to one or more of the intermediate nylon layer **(125)** and the inner polyethylene layer **(135).**

3. The thermotherapeutic pad **(100, 500)** of claim 2, wherein the adhesive **(120, 130)** comprises a polyethylene adhesive.

4. The thermotherapeutic pad **(100, 500)** of any one of claims 1 to 3, wherein the outer polyester layer **(115)** comprises a warp-knitted polyester fabric.

5. The thermotherapeutic pad **(100, 500)** of claim 4, wherein the warp-knitted polyester fabric has a weight of approximately 120 grams per square meter.

6. The thermotherapeutic pad **(100, 500)** of any one of claims 1 to 5, wherein the at least one laminate sheet **(110)** is heat cured.

7. The thermotherapeutic pad **(100, 500)** of any one of claims 1 to 6, wherein the pouch **(105, 505)** is permanently sealed closed by one or more heat bonded seams **(170).**

8. The thermotherapeutic pad **(100, 500)** of any one of claims 1 to 7 further comprising:
at least one strap **(540)** having a fixed end **(545)** affixed to a portion of the pouch **(105, 505),** the at least one strap **(540)** having a length sized to wrap around a body part to undergo thermal therapy to retain the pouch **(105, 505)** against the body part, the at least one strap **(540)** having a fastener **(555)** at a free end opposite the fixed end **(545),** the fastener **(555)** configured to engage the at least one strap **(540)** or the pouch **(105, 505).**

9. The thermotherapeutic pad **(100, 500)** of claim 8 further comprising:
at least one buckle **(160, 560)** for anchoring the at least one strap **(540),** the at least one buckle **(160, 560)** being affixed to another portion of the pouch **(105, 505),** the at least one strap **(540)** anchored by the at least one buckle **(160, 560)** when threaded through and overlapping the buckle **(160, 560).**

10. A method **(1600)** of manufacturing a **re-usable** thermotherapeutic pad **(100, 500),** the method **comprising:**
**permanently** sealing **(1605)** at least one laminate sheet **(110)** to form a pouch (105, 505) having an open top, the at least one laminate sheet **(110)** including an outer polyester layer **(115),** an intermediate nylon layer **(125)** and an inner polyethylene layer **(135);**
loading **(1610)** the pouch **(105, 505)** with a thermotherapeutic composition **(175) comprising a gel material capable of being heated and cooled**; and
closing **(1615)** the pouch **(105, 505)** by permanently sealing the open top;
**whereby the thermotherapeutic pad (100, 500) is adapted to provide hot therapy when the thermotherapeutic composition (175) is heated and cold therapy when the thermotherapeutic composition (175) is cooled.**

11. The method of claim 10, further comprising, before the permanently sealing, forming the at least one laminate by
adhering the intermediate nylon layer **(125)** to the inner polyethylene layer **(135),** and
surface bonding the outer polyester layer **(115)** to the intermediate nylon layer **(125).**

12. The method of claim 11, wherein the forming further comprises heat curing the at least one laminate sheet **(110).**

13. The method of any one of claims 10 to 12, wherein the permanently sealing comprises heat bonding.

14. The method of any one of claims 10 to 13 further comprising:
attaching **(1620)** at least one strap **(540)** at a fixed end **(545)** to a portion of the pouch **(105, 505)**, the at least one strap **(540)** having a length sized to wrap around **a** body part to undergo thermal therapy to retain the pouch **(105, 505)** against the body part, the at least one strap **(540)** having a fastener **(555)** at a free end opposite the fixed end **(545)**, the fastener **(555)** configured to engage the at least one strap **(540)** or the pouch **(105, 505).**

15. The method of claim 14 further comprising:
attaching **(1625)** to another portion of the pouch **(105, 505)**, at least one buckle **(160, 560)** for anchoring the at least one strap **(540)**, the at least one strap **(540)** anchored by the at least one buckle **(160, 560)** when threading the at least one strap **(540)** through and overlapping the at least one buckle **(160, 560).**

## Patentansprüche

1. Wiederverwendbarer thermotherapeutischer Umschlag (100, 500), umfassend:
einen Beutel (105, 505), der aus wenigstens einer Laminatbahn (110) gebildet ist;
wobei die wenigstens eine Laminatbahn (110) eine äußere Polyesterschicht (115), eine Nylon-Zwischenschicht (125) und eine innere Polyethylenschicht (135) umfasst; und
der Beutel (105, 505) permanent dicht verschlossen ist; und
eine thermotherapeutische Zusammensetzung (175), die zuvor in den Beutel (105, 505) gefüllt wurde;
wobei die thermotherapeutische Zusammensetzung (175) ein Gelmaterial umfasst, das erhitzt und gekühlt werden kann, um dann, wenn der Beutel (105, 505) auf einen Körperteil aufgetragen wird, für eine Wärmetherapie zu sorgen, wenn die thermotherapeutische Zusammensetzung (175) erhitzt ist, und für eine Kältetherapie zu sorgen, wenn die thermotherapeutische Zusammensetzung (175) gekühlt ist.

2. Thermotherapeutischer Umschlag (100, 500) gemäß Anspruch 1, weiterhin umfassend einen Kleber (120, 130), der auf die Nylon-Zwischenschicht (125) und/oder die innere Polyethylenschicht (135) aufgetragen ist.

3. Thermotherapeutischer Umschlag (100, 500) gemäß Anspruch 2, wobei der Kleber (120, 130) einen Polyethylenkleber umfasst.

4. Thermotherapeutischer Umschlag (100, 500) gemäß einem der Ansprüche 1 bis 3, wobei die äußere Polyesterschicht (115) ein Polyester-Kettengewirk umfasst.

5. Thermotherapeutischer Umschlag (100, 500) gemäß Anspruch 4, wobei das Polyester-Kettengewirk ein Basisgewicht von ungefähr 120 Gramm pro Quadratmeter aufweist.

6. Thermotherapeutischer Umschlag (100, 500) gemäß einem der Ansprüche 1 bis 5, wobei die wenigstens eine Laminatbahn (110) thermisch ausgehärtet ist.

7. Thermotherapeutischer Umschlag (100, 500) gemäß einem der Ansprüche 1 bis 6, wobei der Beutel (105, 505) durch eine oder mehrere heißgebondete Nähte (170) permanent dicht verschlossen ist.

8. Thermotherapeutischer Umschlag (100, 500) gemäß einem der Ansprüche 1 bis 7, weiterhin umfassend:
wenigstens einen Riemen (540) mit einem fixierten Ende (545), das an einem Teil des Beutels (105, 505) befestigt ist, wobei der wenigstens eine Riemen (540) eine solche Länge aufweist, dass er um einen Körperteil, der eine Thermotherapie erhalten soll, herum gewickelt werden kann, so dass der Beutel (105, 505) gegen den Körperteil gehalten wird, wobei der wenigstens eine Riemen (540) an einem freien Ende gegenüber dem fixierten Ende (545) einen Verschluss (555) aufweist, wobei der Verschluss (555) so konfiguriert ist, dass er an dem wenigstens einen Riemen (540) oder dem Beutel (105, 505) angreifen kann.

9. Thermotherapeutischer Umschlag (100, 500) gemäß Anspruch 8, weiterhin umfassend:
wenigstens eine Schnalle (160, 560) zur Verankerung des wenigstens einen Riemens (540), wobei die wenigstens eine Schnalle (160, 560) an einem anderen Teil des Beutels (105, 505) befestigt ist, wobei der wenigstens eine Riemen (540) durch die wenigstens eine Schnalle (160, 560) verankert ist, wenn er durch die Schnalle (160, 560) gefädelt ist und dieselbe überlappt.

10. Verfahren (1600) zur Herstellung eines wiederverwendbaren thermotherapeutischen Umschlags (100, 500), wobei das Verfahren umfasst:
permanentes Verschließen (1605) wenigstens einer Laminatbahn (110) unter Bildung eines Beutels (105, 505) mit einer offenen Oberseite, wobei die wenigstens eine Laminatbahn (110) eine äußere Polyesterschicht (115), eine Nylon-Zwischenschicht (125) und eine innere Polyethylenschicht (135) umfasst;
Befüllen (1610) des Beutels (105, 505) mit einer thermotherapeutischen Zusammensetzung (175), die ein Gelmaterial umfasst, das erhitzt und gekühlt werden kann; und
Schließen (1615) des Beutels (105, 505) durch permanentes Verschließen der offenen Oberseite;
wodurch der thermotherapeutische Umschlag (100, 500) geeignet ist, für eine Wärmetherapie zu sorgen, wenn die thermotherapeutische Zusammensetzung (175) erhitzt ist, und für eine Kältetherapie zu sorgen, wenn die thermotherapeutische Zusammensetzung (175) gekühlt ist.

11. Verfahren gemäß Anspruch 10, weiterhin umfassend:
vor dem permanenten Verschließen, Bilden des wenigstens einen Laminats durch
Anheften der Nylon-Zwischenschicht (125) an die innere Polyethylenschicht (135); und
Flächenverkleben der äußeren Polyesterschicht (115) mit der Nylon-Zwischenschicht (125).

12. Verfahren gemäß Anspruch 11, wobei das Bilden weiterhin die thermische Aushärtung der wenigstens einen Laminatbahn (110) umfasst.

13. Verfahren gemäß einem der Ansprüche 10 bis 12, wobei das permanente Verschließen Heißverkleben umfasst.

14. Verfahren gemäß einem der Ansprüche 10 bis 13, weiterhin umfassend:
Befestigen (1620) wenigstens eines Riemens (540) mit einem fixierten Ende (545) an einem Teil des Beutels (105, 505), wobei der wenigstens eine Riemen (540) eine solche Länge aufweist, dass er um einen Körperteil, der eine Thermotherapie erhalten soll, herum gewickelt werden kann, so dass der Beutel (105, 505) gegen den Körperteil gehalten wird, wobei der wenigstens eine Riemen (540) an einem freien Ende gegenüber dem fixierten Ende (545) einen Verschluss (555) aufweist, wobei der Verschluss (555) so konfiguriert ist, dass er an dem wenigstens einen Riemen (540) oder dem Beutel (105, 505) angreifen kann.

15. Verfahren gemäß Anspruch 14, weiterhin umfassend:
Befestigen (1625) wenigstens einer Schnalle (160, 560) zur Verankerung des wenigstens einen Riemens (540) an einem anderen Teil des Beutels (105, 505), wobei der wenigstens eine Riemen (540) durch die wenigstens eine Schnalle (160, 560) verankert ist, wenn man den wenigstens einen Riemen (540) durch die wenigstens eine Schnalle (160, 560) fädelt und damit überlappt.

## Revendications

1. Compresse thermothérapeutique réutilisable (100, 500), comprenant :
un sachet (105, 505) formé à partir d'au moins une feuille laminée (110),
ladite au moins une feuille laminée (110) comprenant une couche polyester extérieure (115), une couche nylon intermédiaire (125), et une couche polyéthylène intérieure (135), et
ledit sachet (105, 505) étant fermé hermétiquement de façon permanente, et
une composition thermothérapeutique (175) préchargée dans ledit sachet (105, 505), ladite composition thermothérapeutique (175) comprenant un matériau de gel pouvant être chauffé et refroidi pour procurer, lorsque le sachet (105, 505) est appliqué contre une partie du corps, une thérapie à chaud quand la composition thermothérapeutique (175) est chauffée, et une thérapie à froid quand la composition thermothérapeutique (175) est refroidie.

2. Compresse thermothérapeutique (100, 500) selon la revendication 1, comprenant en outre un adhésif (120, 130) appliqué sur la couche nylon intermédiaire (125) et la couche polyéthylène intérieure (135).

3. Compresse thermothérapeutique (100, 500) selon la revendication 2, dans laquelle ledit adhésif (120, 130) comprend un adhésif polyéthylène.

4. Compresse thermothérapeutique (100, 500) selon l'une quelconque des revendications 1 à 3, dans laquelle ladite couche polyester extérieure (115) comprend un tissu polyester tricoté à chaîne.

5. Compresse thermothérapeutique (100, 500) selon la revendication 4, dans laquelle ledit tissu polyester tricoté à chaîne présente un poids de base d'environ 120 grammes par mètre carré.

6. Compresse thermothérapeutique (100, 500) selon l'une quelconque des revendications 1 à 5, dans laquelle ladite au moins une feuille laminée (110) est durcie à chaud.

7. Compresse thermothérapeutique (100, 500) selon l'une quelconque des revendications 1 à 6, dans laquelle ledit sachet (105, 505) est fermé hermétiquement de façon permanente par un ou plusieurs joints thermocollés (170).

8. Compresse thermothérapeutique (100, 500) selon l'une quelconque des revendications 1 à 7, comprenant en outre :
au moins une bretelle (540) ayant une extrémité fixe (545) attachée sur une partie dudit sachet (105, 505), ladite au moins une bretelle (540) ayant une longueur suffisante pour être enroulée autour d'une partie du corps à soumettre à une thermothérapie pour retenir ledit sachet (105, 505) contre la partie du corps, ladite au moins une bretelle (540) possédant une fermeture (555) à une extrémité libre opposée à ladite extrémité fixe (545), ladite fermeture (555) étant configurée pour venir en prise sur ladite au moins une bretelle (540) ou ledit sachet (105, 505).

9. Compresse thermothérapeutique (100, 500) selon la revendication 8, comprenant en outre :
au moins une boucle (160, 560) pour ancrer ladite au moins une bretelle (540), ladite au moins une boucle (160, 560) étant attachée à une autre partie dudit sachet (105, 505), ladite au moins une bretelle (540) étant ancrée par ladite au moins une boucle (160, 560) lorsqu'elle est enfilée dans ladite boucle (160, 560), se chevauchant avec elle.

10. Procédé (1600) pour fabriquer une compresse thermothérapeutique réutilisable (100, 500), le procédé comprenant les étapes consistant à :
fermer hermétiquement (1605) au moins une feuille laminée (110) de façon permanente pour former un sachet (105, 505) ayant une partie supérieure ouverte, ladite au moins une feuille laminée (110) comprenant une couche polyester extérieure (115), une couche nylon intermédiaire (125), et une couche polyéthylène intérieure (135),
charger (1610) le sachet (105, 505) avec une composition thermothérapeutique (175) comprenant un matériau de gel pouvant être chauffé et refroidi, et
fermer (1615) le sachet (105, 505) en fermant hermétiquement la partie supérieure ouverte de façon permanente,
ladite compresse thermothérapeutique (100, 500) étant adapté pour procurer une thérapie à chaud quand la composition thermothérapeutique (175) est chauffée, et une thérapie à froid quand la composition thermothérapeutique (175) est refroidie.

11. Procédé selon la revendication 10, comprenant en outre, avant la fermeture permanente, l'étape consistant à former ladite au moins une feuille laminée en
adhérant la couche nylon intermédiaire (125) à la couche polyéthylène intérieure (135), et
coller en surface la couche polyester extérieure (115) sur la couche nylon intermédiaire (125).

12. Procédé selon la revendication 11, dans lequel l'étape consistant à former comprend en outre le durcissement à chaud de ladite au moins une feuille laminée (110).

13. Procédé selon l'une quelconque des revendications 10 à 12, dans lequel l'étape consistant à fermer hermétiquement de façon permanente comprend le collage à chaud.

14. Procédé selon l'une quelconque des revendications 10 à 13, comprenant en outre l'étape consistant à :
attacher (1620) au moins une bretelle (540) avec une extrémité fixe (545) sur une partie dudit sachet (105, 505), ladite au moins une bretelle (540) ayant une longueur suffisante pour être enroulée autour d'une partie du corps à soumettre à une thermothérapie pour retenir ledit sachet (105, 505) contre la partie du corps, ladite au moins une bretelle (540) possédant une fermeture (555) à une extrémité libre opposée à ladite extrémité fixe (545), ladite fermeture (555) étant configurée pour venir en prise sur ladite au moins une bretelle (540) ou ledit sachet (105, 505).

15. Procédé selon la revendication 14, comprenant en outre l'étape consistant à :
attacher (1621) sur une autre partie dudit sachet (105, 505) au moins une boucle (160, 560) pour ancrer ladite au moins une bretelle (540), ladite au moins une bretelle (540) étant ancrée par ladite au moins une boucle (160, 560) lorsque ladite au moins une bretelle (540) est enfilée dans ladite boucle (160, 560), se chevauchant avec elle.
